# EUROPÄISCHE PATENTANMELDUNG

(11) **EP 0 626 171 A1**
(43) Veröffentlichungstag der Anmeldung: **30.11.1994**
(21) Anmeldenummer: 93108460.2
(22) Anmeldetag: 26.05.1993
(51) Int. Cl.: A61K 31/65, A61K 47/22, A61K 47/32, A61K 47/00

(54) **Injektionslösung für die intravenöse oder intramuskuläre Verabreichung an Tiere**

(71) Anmelder: Dörnhöfer, Winfried, D-82269 Geltendorf (DE); Embrechts, Erwin, B-2322 Hoogstraten (BE)
(72) Erfinder: Dörnhöfer, Winfried, D-82269 Geltendorf (DE); Embrechts, Erwin, B-2322 Hoogstraten (BE)
(74) Vertreter: Thiel, Christian, Dr. Dipl.-Chem.

(57) **Zusammenfassung**

Die Erfindung betrifft eine Injektionslösung für die intravenöse oder intramuskuläre Verabreichung an Tiere mit einem Gehalt, bezogen auf 100 ml Lösung, von 5 bis 25 g Oxytetracyclin oder Doxycylin, jeweils als Magnesiumkomplex, 5 bis 25 g Polyvinylpyrrolidon, der notwendigen Menge einer anorganischen oder organischen Base zur Einstellung eines pH-Wertes von 5,0 bis 9,5, sowie üblichen Zusatzstoffen in einer wäßrig-organischen Lösemittelphase, in der die Lösemittelphase aus 8 bis 66 g N-Methylpyrrolidon und/oder 2-Pyrrolidon, vorzugsweise in einem Mischungsverhältnis von 92:8 bis 70:30 nach Gewicht, und Wasser besteht, bezogen auf 100 ml Lösung, sowie ein Verfahren zur Herstellung derselben.

## Beschreibung

Die Erfindung betrifft eine Injektionslösung für die intravenöse oder intramuskuläre Verabreichung an Tiere mit einem Gehalt, bezogen auf 100 ml Lösung, von 5 bis 25 g Oxytetracylin oder Doxycyclin, jeweils als Magnesiumkomplex, und 5 bis 25 g Polyvinylpyrrolidon, der notwendigen Menge einer anorganischen oder organischen Base zur Einstellung eines pH-Wertes von 5,5 bis 9,5 sowie üblichen Zusatzstoffen in einer wäßrig-organischen Lösemittelphase. Die Erfindung betrifft ferner ein Verfahren zur Herstellung einer solchen Injektionslösung.

In vielen Ländern sind Tetracyclin-Antibiotika Mittel erster Wahl bei zahlreichen therapeutischen Indikationen im verterinären Bereich. Dabei ist für parenterale Anwendungen Oxytetracyclin das am meisten verwandte Tetracyclin.

Nichtsdestoweniger ist mit dem Einsatz von Tretracyclinen eine Reihe von ernsthaften Problemen verbunden. So ist der parenterale Einsatz durch ihre schlechte Löslichkeit und geringe Stabilität in wäßrige Medien beschränkt. Zudem führen sie bei intramuskulärer oder subkutaner Injektion zu starken Reizungen an der Injektionsstelle. Weiterhin kann die intravenöse Verabreichung zu starken Nebenwirkungen führen, insbesondere auch tödlichen systemischen Nebenwirkungen.

Das Problem der geringen Löslichkeit von Tetracyclinen in Wasser wurde vielfach durch Einsatz von mit Wasser mischbaren Lösungsmitteln und Co-Lösungsmitteln zu lösen versucht. Beispielsweise sind zu nennen 1,2-Propandiol und N,N-Dimethylacetamid, die ihrerseits stark reizend wirken.

Bessere galenische Lösungen wurden durch die Einführung von wassermischbaren und besser verträglichen Pyrrolidonderivaten erzielt, die als Lösungsmittel oder Co-Lösungsmittel in wäßrigen parenteralen Tetracyclinzubereitungen eingesetzt wurden. Hier sind 2-Pyrrolidon, N-Methylpyrrolidon und 2-Hydroxyethylpyrrolidon zu nennen.

Der Vorteil der vorstehend beschriebenen Oxytetracyclinformulierungen liegt darin, daß mit einer einzigen intramuskulären Injektion einer Dosis von 20 mg pro kg Körpergewicht ein antimikrobiell wirksamer Serumsspiegel erzeugt wird, der bis zu 72 Stunden nach der Injektion anhält - sogenannte Long-acting-Formulierungen (Oxytetracyclin LA).

Für die parenterale, perorale und lokale Verabreichung geeignete Lösungen von Oxytetracyclinmagnesiumkomplexen in bis zu 25 %igem wäßrigem Polyvinylpyrrolidon mit einem Oxytetracyclingehalt von bis zu 15 % sind aus GB-A-1 131 007 bekannt. Die dort beschriebenen Lösungen haben einen pH-Wert im Bereich von 8,0 bis 9,5. Der pH-Wert wird mit Hilfe von Natriumhydroxid, Ammoniak, Ethanolamin oder Ethylendiamin eingestellt. Derartige Lösungen sind relativ gut verträglich, haben aber den Nachteil, daß eine höhere Konzentration als 15 % nicht einstellbar ist und zudem die für eine problemlose Verabreichung zuträglichen Viskositätswerte überschritten werden.

Aus DE-A-26 15 140 ist eine Wirkstofformulierung für die topische transdermale Verabreichung mit einem Trägersystem bekannt, das aus N-Methylpyrrolidon und 2-Pyrrolidon in einem Verhältnis von 1:4 bis 4:1 besteht. Das Trägersystem in der dort beschriebenen Zusammensetzung soll den Durchtritt des auf die Haut aufgebrachten Wirkstoffs durch die Haut fördern. Eine Verabreichung derartiger Formulierungen durch Injektion ist nicht vorgesehen.

Die DE-A-26 59 152 beschreibt für die Injektion geeignete Formulierungen eines Tetracyclinkomplexes in wäßrigem 2-Pyrrolidon, das Polyvinylpyrrolidon enthalten kann. Die dort beschriebenen Lösungen haben sich nach jüngeren Untersuchungen als ausgesprochen gewebereizend erwiesen. Die Injektionen sind für die Tiere häufig schmerzhaft und führen zu unerwünschten Nekrosen. Die Resorptionszeiten sind relativ lang und die damit erzielten Serumsspiegel verbesserungswürdig. Hohe Rückstandswerte im Gewebe bedingen unerwünschte Wartezeiten. Aufgrund der starken Reizung kommt eine intravenöse Verabreichung dieser Injektionslösungen nicht in Frage.

EP-A-0 096 942 beschreibt eine Oxytetracylinzusammensetzung, bei der 10 bis 30 Gew.-Teile Oxytetracyclin in Form eines Erdalkalikomplexes und 2,5 bis 10 Gew.-Teile Polyvinylpyrrolidon zusammen mit Antioxidantien und Basen bei einem pH-Wert von 6,0 bis 9,5 in wäßrigem N-Methylpyrrolidon gelöst sind. Die dort beschriebenen Lösungen sind für die intramuskuläre Injektion bestimmt und zeichnen sich durch eine verbesserte Verträglichkeit aus. Nachteilig bei diesen Lösungen ist aber immer noch die Tendenz zu Gewebereizungen.

EP-B-0 271 374 beschreibt ferner die Verwendung von N-Hydroxyethyl-2-pyrrolidon als Lösungsvermittler für Tetracycline und insbesondere für i.v. zu verabreichendes Doxycyclin. Die Doxycyclinpräparate führen aber zu ausgeprägten Herz-Kreislauf-Komplikationen.

Eine weitere Formulierung eines Oxytetracyclins in einem wäßrig organischen Lösungsmittel wurde unter der Bezeichnung Tridox vertrieben. Als organische Lösungsmittelkomponente dient eine Mischung aus etwa gleichen Teilen N-Methylpyrrolidon und 2-Pyrrolidon. Die Mischung enthält Polyvinylpyrrolidon zur Erhöhung der Löslichkeit und zur Verbesserung der Verträglichkeit. Auch diese Formulierung, die für die intramuskuläre Verabreichung bestimmt ist, führt an der Injektionsstelle zu erheblichen Reizungen. Die Resorption und die Serumspiegel sind, auch aufgrund der Reizzustände, nicht optimal.

In allen diesen Formulierungen wird das jeweilige Tetracyclin zunächst an ein Erdalkalimetallion komplexiert, wonach die Formulierung auf einen zuträglichen pH-Wert eingestellt wird. Beides dient der Verbesserung der chemischen und physikalischen Stabilität der Tetracyclinlösung. Dabei ist zu beachten, daß der optimale pH-Wert für die verschiedenen Tetracyclin-Derivate sehr unterschiedlich sein kann und beispielsweise für Oxytetracyclin zwischen 7,5 und 9,5 liegt und für Doxycyclin zwischen 5 und 7.

Weitere Formulierungen, die in den wesentlichen Punkten den oben aufgezeigten entsprechen, sind in der Vergangenheit bekanntgeworden und spiegeln die langjährige Forschungstätigkeit auf diesem Gebiet wieder. Trotz dieser intensiven Forschungstätigkeit ist es aber bislang nicht gelungen, die präparatbedingten Nebenwirkungen von Tetracyclinformulierungen und insbesondere solchen mit Oxytetracylin und Doxycyclin vollständig in den Griff zu bekommen. Als präparatbedingte Nebenwirkungen gelten bei der intramuskulären Verabreichung von Oxytetracyclinpräparaten Reizungen an der Injektionsstelle.

Häufigste Nebenwirkung sind örtliche Reizungen an der Injektionsstelle, die zu Nekrosen und Abkapselungen führen und eine Verminderung oder Verzögerung der Resorption des Wirkstoffes bewirken können. Als Folge ergeben sich auch lange Wartezeiten bis zum Schlachtzeitpunkt und teilweise ein Verwertungsverbot für das um die Injektionsstelle liegende Gewebe.

Amtstierärzten und Inspektoren von Schlachthäusern ist dieses Problem sehr vertraut. Es wurde gefolgert, daß Zubereitungen, die zu starken Nekrosen im Muskelgewebe führen, den heutigen Anforderungen an die veterinärmedizinische Praxis nicht mehr genügen (Vet. Quarterly, 1984, 6, 2, 80 - 84; ibid., 1990, 12, 3, 129 - 138). Nach diesen Veröffentlichungen ist nur eine einzige injizierbare Oxytetracyclinzubereitung hinreichend reizarm eingestellt, nämlich die gemäß o.g. GB-A-1 131 007. Bei diesen Zubereitungen sind die Muskelschäden an der Injektionsstelle gering und sind nach einigen Wochen vollständig verschwunden. In der Praxis hat sich aber gezeigt, daß diese Lösungen aufgrund ihrer hohen Viskosität nur bis zu einem Oxytetracyclingehalt von bis zu 10 % injizierbar sind.

Nach der intravenösen Verabreichung von Tetracyclinantibiotika auftretende Nebenwirkungen wurden ebenfalls gründlich untersucht. Außer der tetracyclinbedingt auftretenden hepatotoxischen und tödlichen nephrotoxischen wurden lebensbedrohende Herz-Kreislauf-Effekte beobachtet, die einerseits auf die Chelierung von freien Calciumionen im Serum einerseits und in den Formulierungen vorhandenes Propylenglykol und Polyvinylpyrrolidon andererseits zurückgeführt wurden (JAVMA, 1984, 185, 793-795; Bov. Pract., 1979, 14, 29-35 & 37-41; Am. J. vet. Res., 1979, 40, 783-791; ibid., 1981, 42, 1371-1377; ibid., 1985, 45, 1658-1659, J. vet. Pharm. Therap., 1981, 4, 15-25; Zbl. Vet. Med. A, 1980, 27, 228-237).

Insgesamt wurde die Erfahrung gemacht, daß die parenterale Verabreichung von Oxytetracyclin- und Doxycyclinpräparaten sehr sorgfältig gehandhabt und überwacht werden muß, um Komplikationen zu vermeiden oder gering zu halten. Bei der intravenösen Verabreichung besteht ein relativ hohes Risiko, daß tödliche Nebenwirkungen auftreten.

Angesichts dessen ist Aufgabe der Erfindung die Bereitstellung von oxytetracyclin- und doxycyclinhaltigen Injektionslösungen, die eine verminderte Tendenz zur Erzeugung von Reizzuständen bei intramuskulärer Injektion aufweisen und ein geringes Risiko von Herz-Kreislauf-Problemen bei intravenöser Injektion. Zugleich sollen diese Lösungen gegenüber den bisher bekannten Präparaten wenigstens gleichwertige Serumspiegel und eine vollständige Ausscheidung aus dem Körper gewährleisten.

In der Pharmakokinetik ist allgemein anerkannt, daß Formulierungen mit guter lokaler Verträglichkeit auch optimale Resorptionscharakteristiken und eine gute Bioverfügbarkeit ergeben.

Diese Aufgabe wird mit einer Injektionslösung der eingangs genannten Art gelöst, bei der die Lösungsmittelphase aus 8 bis 66 g N-Methylpyrrolidon und/oder 2-Pyrrolidon sowie Wasser besteht, bezogen auf 100 ml Lösung.

Vorzugsweise beträgt das Verhältnis N-Methylpyrrolidon/2-Pyrrolidon 92:8-70:30.

Zur Erhöhung der Verträglichkeit der erfindungsgemäßen Injektionslösungen hat es sich als vorteilhaft erwiesen, das Polyvinylpyrrolidon in Wasser unter Druck bei einer Temperatur von mehr als 115°C in Lösung zu bringen. Der Zeitraum dieses Autoklavierungsprozesses soll wenigstens etwa 15 min betragen, zweckmäßigerweise wenigstens etwa 30 min. Zur Stabilisierung und Vermeidung von Verfärbungen kann der Lösung während des Autoklavierungsprozesses Natriummetabisulfit, etwa in einer Menge bis zu 0,5 Gew.-% und vorzugsweise von etwa 0,1 Gew.%, bezogen auf das Polyvinylpyrrolidon, zugesetzt werden.

Das zum Einsatz kommende Polyvinylpyrrolidon hat zweckmäßigerweise ein Molekulargewicht im Bereich von 5.000 bis 30.000. Besonders bevorzugt sind Molekulargewichte von 10.000 bis 17.000, angegeben. Geeignete K-Werte sind beispielsweise K12 und K17. Bevorzugte Polyvinylpyrrolidone sind Kollidone der Fa. BASF und Plasdone der Fa. GAF.

Es wurde festgestellt, daß in bekannten Formulierungen die immanente gewebereizende Wirkung von Tetracyclinen durch die gewählten Ausgangsmaterialien und Herstellungsverfahren zusätzlich erhöht wird und häufig erst dadurch kritische Werte erreicht. Durch Auswahl geeigneter Ausgangsstoffe und geeignete Maßnahmen bei der Formulierung kann somit erreicht werden, daß die Zubereitungen reizarm und verträglich eingestellt werden können.

Der gewünschte pH-Wert im Bereich von 5,0 bis 9,5 kann mit einer üblichen anorganischen oder organischen Base eingestellt werden, beispielsweise Natriumhydroxid, Ammoniak, Mono- oder Diethanolamin oder anderen üblichen niedermolekularen Aminen. Besonders bevorzugt ist aber die Verwendung basischer Aminosäuren, wie L-Arginin, L-Lysin oder L-Ornithin oder anderer Aminosäuren mit wenigstens zwei Aminofunktionen, da sich gezeigt hat, daß die Verwendung von Natriumhydroxid oder Ethanolaminen ein erhebliches zusätzliches Reizpotential ausgeht. Die Verwendung basischer Aminosäuren ist geeignet, die Verträglichkeit der erfindungsgemäßen Zubereitungen weiter zu erhöhen. Es kann dabei aber zweckmäßig sein, zusätzlich konventionelle Basen in geringeren Mengen zuzusetzen, um die pH-Einstellung zu erleichtern oder das Ausfallen der Aminosäure wegen Überschreitung der Löslichkeit zu vermeiden.

Die Verträglichkeit kann weiterhin durch die Verwendung chloridionenfreier Ausgangsmaterialien verbessert werden. Die hier verwandten Begriffe "chloridionenfrei" bzw. "im wesentlichen chloridionenfrei" bedeuten, daß der Chloridionenanteil sich in einer Größenordnung bewegt, die keine physiologischen Reizzustände mehr hervorrufen kann.

Im Grunde genommen können die erfindungsgemäßen Lösungen aus beliebigen, auch chloridhaltigen Ausgangsstoffen hergestellt werden. Für die chloridionenfreie Einstellung müssen dann Maßnahmen getroffen werden, unzuträgliche Chloridmengen aus der fertigen Lösung zu entfernen. Dies kann beispielsweise dadurch erfolgen, daß man die Magnesiumkomplexe zwar ausgehend von chloridionenhaltigen Ausgangsstoffen, wie Hydrochloriden und Magnesiumchlorid, darstellt, die dabei anfallenden Chloride jedoch, beispielsweise durch Kristallisation, abtrennt. Um ein im wesentlichen chloridionenfreies Produkt zu erhalten, ist es jedoch bevorzugt, ein chloridionenfreies Magnesiumsalz zu verwenden, insbesondere Magnesiumoxid. Oxytetracyclin und Doxycyclin werden vorzugsweise in Form ihrer Hydrate oder als freie Base eingesetzt. Es können auch chloridionenfreie Salze verwandt werden, deren Verträglichkeit und Reizarmut bekannt ist. Bevorzugt sind chloridionenfreie Ausgangsmaterialien.

Die erfindungsgemäßen Injektionslösungen werden vorzugsweise auf eine Spritzviskosität von weniger als 25 cps und insbesondere weniger als 70 cps eingestellt. Besonders bevorzugt ist eine Spritzviskosität von weniger als 50 cps.

Als besonders geeignet für die intramuskuläre Verabreichung haben sich Oxytetracyclinformulierungen mit einem Oxytetracyclingehalt von etwa 20 g auf 100 ml Lösung erwiesen. Eine solche Formulierung enthält in der Lösungsmittelphase vorzugsweise 20 bis 60 g N-Methylpyrrolidon und 2-Pyrrolidon in dem angegebenen Mischungsverhältnis. Zur Stabilisierung der Lösung und Erhöhung der Verträglichkeit werden 5 bis 15 g Polyvinylpyrrolidon auf 100 ml Lösung zugesetzt.

Vorzugsweise ist dabei das Gewichtsverhältnis von N-Methylpyrrolidon zu 2-Pyrrolidon kleiner als 90:10 und größer als 80:20 und insbesondere etwa 5:1 bis 4:1. Es hat sich herausgestellt, daß bei Einhaltung dieser Grenzen ein Optimum an Lösungseffekt bei guter Spritzbarkeit und guter Verträglichkeit erzielt wird. Zugleich garantiert die Mischung eine gute Resorption des intramuskulär gespritzten Arzneimittels, so daß frühzeitig die gewünschten hohen Serumspiegel erreicht werden. Geweberückstände werden binnen kurzer Zeit vollständig abgebaut, so daß sich eine Verkürzung der bisher einzuhaltenden Wartezeiten ergibt.

Zahlreiche bekannte Oxytetracyclin-LA-Formulierungen enthalten Oxytetracyclin als Hydrochlorid oder Base, komplexiert an Magnesium, das als MgO oder MgCl₂ zugesetzt wurde. Der End-pH-Wert dieser Formulierungen wird mit organischen Basen eingestellt, in den meisten Fällen mit Mono- oder Diethanolamin. In anderen Fällen wird Salzsäure zugesetzt. Niedrige Polyvinylkonzentrationen, etwa 5 Gew.-%, werden zur Erhöhung der örtlichen Verträglichkeit zugesetzt, wobei die Menge durch die Viskosität der jeweiligen Formulierung begrenzt ist. Formulierungen mit 7 bis 10 % Polyvinylpyrrolidon in der Lösung sind zwar vorgeschlagen worden, haben sich aber wegen ihrer hohen Viskositätswerte in der Praxis nicht durchsetzen können.

Mit den erfindungsgemäßen Formulierungen können demgegenüber eine Reihe von Verbesserungen und Vorteilen erzielt werden. Es hat sich herausgestellt, daß die lokale Reizung an der Injektionsstelle das Ergebnis von verschiedenen Parametern ist. Dabei wurde gefunden, daß die Gegenwart von MgCl₂ in der Lösung ein wichtiger Faktor ist. In der Formulierung verbliebenes MgCl₂ kann auf eine unvollständige Komplexierung oder auf die Zugabe von HCl zur Einstellung des pH-Werts zurückzuführen sein. Aus diesem Grunde sollte der Einsatz von Chloridionen bei der Formulierung der Mittel vermieden werden und, zur Vermeidung von unerwünschten Nebenreaktionen oder Nebenprodukten aufeinander abgestimmte Mengen der verschiedenen Reagenzien eingesetzt werden, insbesondere was das Oxytetracyclin und das Magnesiumsalz betrifft. Bevorzugt sind äquimolare Mengen an Tetracyclin und Magnesiumsalz oder eine Abweichung von maximal ± 12 % von der Äquimolarität.

Örtliche Irritationen an der Injektionsstelle werden weiterhin durch das Fortlassen von stark reizenden Exzipienten vermieden, wie beispielsweise organischen Basen zur Einstellung des pH-Wertes, etwa Mono- und Diethanolamin. Studien von T. Sado zeigen die starke lokale Reizwirkung von Ethanolaminen bei Sulfonamiden (Nippon Nogeikagaku Kaishi, 1961, 35, 1164). Es wurde gefunden, daß basische Aminosäuren, wie L-Arginin, L-Lysin oder L-Ornithin geeignete Substitutionsprodukte sind.

Es ist allgemein bekannt, daß Polyvinylpyrrolidon die lokale Verträglichkeit von Tetracyclinen verbessert. Polyvinylpyrrolidon erhöht jedoch die Viskosität und verschlechtert die Spritzbarkeit der Formulierung. Durch eine Autoklavierung der zur Formulierung verwandten wäßrigen Polyvinylpyrrolidonlösung kann die Spritzbarkeit auch von hochkonzentrierten Lösungen verbessert werden, da sich nach dem Autoklavierungsschritt ein um 10 bis 20 % verbesserter Viskositätswert für die Lösungen ergibt. Verantwortlich hierfür sind vermutlich Hydrolysereaktionen. Die Stabilität der Polyvinylpyrrolidonlösung kann durch die Zugabe eines Antioxidants, wie Natriumetabisulfit bei der Autoklavierung verbessert werden.

Schließlich wurde gefunden, daß die Mischung zwei verschiedener Lösungsmittel vom Pyrrol-Typ die Resorptionscharakteristiken gegenüber der Verwendung der jeweiligen Lösungsmittel allein weiter verbessert. Hinweise auf diesen Effekt finden sich bereits in der DE-A-2 615 140, dort aber für den Fall transdermaler Formulierungen. Es wurde jetzt gefunden, daß entsprechendes auch für subkutan oder intramuskulär zu verabreichende Formulierungen gilt. 2-Pyrrolidon hat einen stärker verzögernden Effekt als N-Methylpyrrolidon, wobei ein Verhältnis zwischen 1:4 und 1:5 die besten Verfügbarkeitsergebnisse liefert.

Die ausgezeichneten pharmakokinetischen Eigenschaften der erfindungsgemäßen Zubereitungen wurden durch Untersuchung der klassischen Serumkurven bestätigt. Die Serum-Kreatinin-Phosphokinasewerte ergaben eine gute lokale Verträglichkeit, die sich in der Praxis bestätigt hat.

Doxycyclinformulierungen für die intravenöse Verabreichung haben vorzugsweise einen Doxycyclingehalt von etwa 10 g auf 100 ml Lösung bei einem Gehalt von 8 bis 20 g N-Methylpyrrolidon und/oder 2-Pyrrolidon in 100 ml Lösung. Die Verträglichkeit derartiger Injektionslösungen wird durch einen relativ hohen Polyvinylpyrrolidongehalt von 15 bis 25 g pro 100 ml Lösung gefördert. Hier können neben Formulierungen, die N-Methylpyrrolidon und 2-Pyrrolidon in den für Oxytetracyclin genannten Verhältnissen auch solche mit 2-Pyrrolidon als alleinigem organischen Lösungsmtitel eingesetzt werden.

Aufgrund seiner hohen Lipophilität und seiner starken Gewebeaffinität wird Doxycyclin bei intramuskulärer oder subkutaner Injektion sehr ungleichmäßig und auf eine nicht vorhersehbare Weise resorbiert. In klinischen Versuchen wurde jedoch festgestellt, daß intravenös in recht hohen Dosen verabreichtes Doxycyclin allen anderen Antibiotika bei der Behandlung ernster und kritischer Infektionen der Atemorgane bei jungen Kälbern überlegen ist. Die jetzt erarbeiteten erfindungsgemäßen Formulierungen haben sich als außergewöhnlich gut verträglich erwiesen.

Selbst von Tieren mit schweren Infektionen wurden diese Formulierungen für die intravenöse Verabreichung gut toleriert.

Derzeit verfügbare Doxycyclinformulierungen beruhen darauf, daß Doxycyclin mittels Polyvinylpyrrolidion oder mit einem Lösungsmittel vom 2-Pyrrolidontyp in wäßrige Lösung gebracht wird. Die gleichzeitige Verwendung von Polyvinylpyrrolidon und 2-Pyrrolidon ist bislang nicht bekanntgeworden. Grund hierfür ist die Tendenz zur Gelbildung bei solchen Formulierungen.

Es wurde jetzt gefunden, daß die gleichzeitige Verwendung von 2-Pyrrolidon und Polyvinylpyrrolidon in hohen Konzentrationen in einer Formulierung zu intravenösen Zubereitungen führt, die bei guter Spritzviskosität leicht verabreichbar sind und von den damit behandelten Tieren gut vertragen wird. Dosen von 40 bis 50 mg pro kg Körpergewicht konnten verabreicht werden, ohne daß es zu den bekannten Nebenwirkungen gekommen ist. Dabei wurde die pH-Einstellung mit Ascorbinsäure und basischen Aminosäuren vorgenommen, wie beschrieben.

Die erfindungsgemäßen Injektionslösungen enthalten übliche Zusatzstoffe zur Stabilisierung, Konservierung, Erhöhung der Spritzbarkeit, etc. Geeignete Konservierungsmittel sind beispielsweise Ascorbinsäure, Hydroxylmethansulfinsäure, Magnesiumformaldehydsulfoxylat oder das entsprechende Natriumsalz, Natriummetabisulfit oder Natriumsulfit.

Erfindungsgemäße Formulierungen werden vorzugsweise nach einem Verfahren hergestellt, bei dem in einem ersten Schritt die erforderliche Menge Polyvinylpyrrolidon in Wasser gelöst und über einen Zeitraum von wenigstens 15 min bei einer Temperatur von mehr als 115°C autoklaviert wird, diese Lösung nach dem Abkühlen in einem zweiten Schritt mit N-Methylpyrrolidon und/oder 2-Pyrrolidon, Stabilisierungsmittel und einem Magnesiumsalz, jeweils in den erforderlichen Mengen, versetzt und bis zur Homogenität gerührt wird, danach in einem dritten Schritt die erforderliche Menge Oxytetracyclin, Doxycyclin oder eines Derivats davon eingerührt wird und in einem letzten Schritt durch Zufügen einer Base der gewünschte pH-Wert eingestellt wird.

Die Erfindung wird durch nachstehende Beispiele näher erläutert.

### Beispiel 1

25,0 kg Polyvinylpyrrolidon 12PF werden in 130 l destilliertem Wasser unter Zusatz von 2,5 g Natriummetabisulfit gelöst und bei 125°C 2 h autoklaviert. Nach dem Abkühlen der Lösung werden 200 l N-Methylpyrrolidon, 40 l 2-Pyrrolidon, 2,0 kg Magnesiumformaldehydsulfoxylat und 9,15 kg Magnesiumoxid in die Lösung eingerührt. Hierzu werden unter kräftigem Rühren 90 kg, berechnet als freie Base, Oxytetracyclin als Dihydrat gegeben. Es wird gerührt, bis sich eine homogene Lösung eingestellt hat. 10,00 kg Monoethanolamin werden hinzugefügt, danach 10 kg Oxytetracyclin als Dihydrat und 8,0 kg Monoethanolamin, wonach mit destilliertem Wasser auf 500 l aufgefüllt wird. Es ergibt sich eine Formulierung mit einem Oxytetracyclingehalt von 20 g pro 100 ml mit einer spezifischen Dichte von etwa 1,145, eine Spritzviskosität von etwa 30 cps (Kapillarmethode) die mit einer Spritzzeit von etwa 10 s verabreicht werden kann. Der Polyvinylpyrrolidongehalt beträgt 5 g/ml, das Volumenverhältnis von N-Methylpyrrolidon zu 2-Pyrrolidon 83,3 zu 16,7.

Die Formulierung eignet sich besonders für die intramuskuläre Verabreichung und wird gut vertragen. Die Verträglichkeit kann durch eine Erhöhung des Polyvinylpyrrolidongehalts auf 40 kg (8 g/100 ml) noch erhöht werden.

### Beispiel 2

Eine Injektionslösung mit einem Gehalt von 10 % Oxytetracyclin für die intramuskuläre Verabreichung wird aus folgenden Bestandteilen analog zu Beispiel 1 hergestellt:

| | |
|---|---|
| Oxytetracyclindihydrat | 107,82 g |
| eq. Oxytetracyclin | 100,00 g |
| Magnesiumoxid | 8,85 g |
| Hydroxylmethansulfinsäure, Natriumsalz.2 H₂O | 2,00 g |
| N-Methylpyrrolidon | 206,00 g |
| 2-Pyrrolidon | 44,40 g |
| Poly-(1-vinyl-2-pyrrolidon) | 150,00 g |
| 2-Aminoethanol | 21,16 g |
| Wasser für Injektionszwecke q.s.ad | 1,00 l. |

### Beispiel 3

Eine Doxycyclinformulierung für die intravenöse Injektion mit einem Doxycyclingehalt von 10 g pro 100 ml wird analog zu Beispiel 1 hergestellt. Es wurden die nachstehend aufgeführten Bestandteile verarbeitet:

| | |
|---|---|
| Doxycyclinhyclat | 121,17 g |
| eq. Doxycyclin | 100,00 g |
| Magnesiumchlorid | 94,50 g |
| Ascorbinsäure | 2,00 g |
| Poly-(1-vinyl-2-pyrrolidon) | 200,00 g |
| 2-Aminoethanol | 28,00 g |
| 2-Pyrrolidon | 25,00 g |
| N-Methylpyrrolidon | 70,00 g. |
| Wasser für Injektionszwecke q.s. ad | 1,00 l. |

Die erhaltene Lösung hat eine Spritzviskosität von etwa 50 cps (Kapillarmethode). Sie ist bei intravenöser Verabreichung an Rinder ausgesprochen gut verträglich, was auf den hohen Gehalt von 20 g pro 100 ml Polyvinylpyrrolidon zurückzuführen ist.

### Beispiel 4

| | |
|---|---|
| Oxytetracyclindihydrat | 21,60 g |
| entsprechend 20 g Oxytetracyclin Magnesiumoxid | 1,60 g |
| Polyvinylpyrrolidon (K 17) | 8,00 g |
| Natriummetabisulfit | 0,008 g |
| 2-Pyrrolidon | 8,88 g |
| N-Methylpyrrolidon | 41,20 g |
| Natriumformaldehydsulfoxylat | 0,40 g |
| L-Arginin | 3,25 g |
| Wasser für Injektionszwecke ad | 100,00 ml. |

Das Pyrrovinylpyrrolidon wird mit Natriumbetabisulfit in etwa 25 ml Wasser gelöst und bei 121°C 30 min. im Autoklaven behandelt. Nach dem Abkühlen auf Raumtemperatur wird das 2-Pyrrolidon und das N-Methylpyrrolidon zugemischt, danach das Magnesiumoxid zugegeben und gerührt, bis eine homogene Aufschlämmung erhalten wird.

Im Anschluß daran wird das Natriumformaldehydsulfoxylat und das L-Arginin zugesetzt und auf 50°C erwärmt. Unter kontinuierlichem Rühren wird das Oxytetracyclindihydrat langsam zugegeben, wonach vorsichtig erwärmt wird, bis das gesamte Oxytetracyclin vollständig gelöst und die Lösung klar geworden ist. Es wird auf Raumtemperatur abgekühlt und mit Wasser für Injektionszwecke auf das Endvolumen eingestellt. Nach der Sterilisation durch einen Bakterien zurückhaltenden Filter wird die Lösung unter Stickstoff in Ampullen abgefüllt.

Der pH-Wert der Lösung war 8,90 und die Viskosität bei 25°C 45 cSt.

### Beispiel 5

Eine Formulierung gemäß Beispiel 4 wurde hergestellt, wobei jedoch anstelle von 3,25 gl Arginin 2,00 g L-Arginin und 0,3 ml Monoethanolamin verwandt wurden. Es ergab sich ein pH-Wert von 8,80, die Viskosität bei 25°C war 42 cSt.

### Beispiel 6

Eine Formulierung gemäß Beispiel 4 wurde hergestellt, wobei jedoch anstelle von L-Arginin 0,025 g L-Cystein als Hydrochlorid (Oxidationsschutz) und 3,25 g L-Arginin verwandt wurden. Es ergab sich ein pH-Wert von 8,77. Die Viskosität bei 25°C betrug 44 cSt.

### Beispiel 7

Folgende Bestandteile wurden zu einer Oxytetracyclinformulierung verarbeitet, wobei die in Beispiel 4 beschriebene Vorgehensweise verwandt wurde:

| | |
|---|---|
| Oxytetracyclindihydrat | 21,60 g |
| entsprechend 20 g Oxytetracyclin Magnesiumoxid | 1,60 g |
| Polyvinylpyrrolidon (K 17) | 15,00 g |
| Natriummetabisulfit | 0,008 g |
| 2-Pyrrolidon | 8,88 g |
| N-Methylpyrrolidon | 41,20 g |
| Natriumformaldehydsulfoxylat | 0,40 g |
| L-Arginin | 3,25 g |
| Wasser für Injektionszwecke ad | 100,00 ml. |

Es ergab sich eine Lösung mit einem pH-Wert von 8,77. Die Viskosität bei 25°C betrug 108 cSt.

### Beispiel 8

Folgende Bestandteile wurden zu einer Doxycyclin-Formulierung verarbeitet:

| | |
|---|---|
| Doxycyclinhyclat | 11,832 g |
| entsprechend 10 g Doxycyclin Magnesiumchlorid X 6 H₂O | 9,440 g |
| Polyvinylpyrrolidon (K 17) | 20,000 g |
| Natriummetabisulfit | 0,020 g |
| 2-Pyrrolidon | 10,544 g |
| Ascorbinsäure | 0,200 g |
| Monoethanolamin ad pH 5,7 ± | 2,800 ml |
| Wasser für Injektionszwecke ad | 100,000 ml |

Das Polyvinylpyrrolidon wurde mit Natriummetabisulfit in etwa 50 ml Wasser für Injektionszwecke gelöst und bei 121°C 30 min. erhitzt. Nach dem Abkühlen auf Raumtemperatur wurde das 2-Pyrrolidon in die Lösung eingemischt, nachfolgend das Monoethanolamin, daß Magnesiumchlorid und die Ascorbinsäure. Das Doxycyclinhyclat wurde unter kontinuierlichem Rühren langsam zugegeben und die Mischung bis zur vollständigen Komplexierung des Doxycyclins unter Stickstoff gerührt. Anschließend wurde steriles Wasser für Injektionszwecke bis zum Endvolumen zugesetzt, steril filtriert und unter Stickstoff in Ampullen abgefüllt. Es ergab sich ein pH-Wert von 5,66. Die Endviskosität war 23 cSt bei 25°C.

### Beispiel 9

Eine Doxycyclinformulierung wurde gemäß Beispiel 8 hergestellt, wobei anstelle von Monoethanolamin L-Arginin in einer Menge von ± 3,200 g zugesetzt wurde. Es ergab sich ein pH-Wert von 5,69 für die fertige Lösung und eine Viskosität von 26 cSt bei 25°C.

## Patentansprüche

1. Injektionslösung für die intravenöse oder intramuskuläre Verabreichung an Tiere mit einem Gehalt, bezogen auf 100 ml Lösung, von 5 bis 25 g Oxytetracyclin oder Doxycylin, jeweils als Magnesiumkomplex, 5 bis 25 g Polyvinylpyrrolidon, der notwendigen Menge einer anorganischen oder organischen Base zur Einstellung eines pH-Wertes von 5,0 bis 9,5, sowie üblichen Zusatzstoffen in einer wäßrig-organischen Lösemittelphase, dadurch gekennzeichnet, daß die Lösemittelphase aus 8 bis 66 g N-Methylpyrrolidon und/oder 2-Pyrrolidon, vorzugsweise in einem Mischungsverhältnis von 92:8 bis 70:30 nach Gewicht, und Wasser besteht, bezogen auf 100 ml Lösung.

2. Injektionslösung nach Anspruch 1, hergestellt unter Verwendung einer Polyvinylpyrrolidonlösung in Wasser, die durch Autoklavieren vorzugsweise bei über 115°C für wenigstens 15 min erhalten wurde.

3. Injektionslösung nach Anspruch 2, dadurch gekennzeichnet, daß die Polyvinylpyrrolidonlösung durch Autoklavieren in Gegenwart von Natriummethabisulfid in einer Menge von bis zu 0,5 Gew.-%, vorzugsweise von etwa 0,1 Gew.-%, bezogen auf das Polyvinylpyrrolidon, erhalten wurde.

4. Injektionslösung nach einem der Ansprüche 1 bis 3, dadurch gekennzeichnet, daß sie eine basische Aminosäure, insbesondere L-Arginin, L-Lysin und L-Ornithin, enthält.

5. Injektionslösung nach einer der vorstehenden Ansprüche, dadurch gekennzeichnet, daß das Polyvinylpyrrolidon ein Molekulargewicht von 5.000 bis 30.000 hat, vorzugsweise von 10.000 bis 17.000.

6. Injektionslösung nach einem der vorstehenden Ansprüche, dadurch gekennzeichnet, daß sie im wesentlichen frei von Chloridionen ist.

7. Injektionslösung nach einem der vorstehenden Ansprüche mit einer Spritzviskosität von weniger als 125 cps, vorzugsweise weniger als 70 cps.

8. Injektionslösung nach einem der Ansprüche 1 bis 7 für die intramuskuläre Verabreichung mit einem Oxytetracyclingehalt von 20 g auf 100 ml Lösung.

9. Injektionslösung nach Anspruch 8, gekennzeichnet durch einen Gehalt von 20 bis 60 g N-Methylpyrrolidon und 2-Pyrrolidon in einem Gewichtsverhältnis von 90:10 bis 80:20, insbesondere 5:1 bis 4:1, auf 100 ml Lösung.

10. Injektionslösung nach Anspruch 8 oder 9, gekennzeichnet durch einen Gehalt von 5 bis 15 g Polyvinylpyrrolidon auf 100 ml Lösung.

11. Injektionslösung für die intravenöse Verabreichung nach einem der Ansprüche 1 bis 7, gekennzeichnet durch einen Doxycyclingehalt von etwa 10 g pro 100 ml Lösung.

12. Injektionslösung nach Anspruch 11, gekennzeichnet durch einen Gehalt von 8 bis 20 g N-Methylpyrrolidon und/oder 2-Pyrrolidon auf 100 ml Lösung.

13. Injektionslösung nach Anspruch 12, dadurch gekennzeichnet, daß sie nur 2-Pyrrolidon als organisches Lösungsmittel enthält.

14. Injektionslösung nach Anspruch 12, dadurch gekennzeichnet, daß sie N-Methylpyrrolidon und 2-Pyrrolidon in einem Gewichtsverhältnis von 5:1 bis 4:1 enthält.

15. Injektionslösung nach einem der Ansprüche 11 bis 14, gekennzeichnet durch einen Gehalt von 15 bis 25 g Polyvinylpyrrolidon auf 100 ml Lösung.

16. Verfahren zur Herstellung einer Injektionslösung nach einem der vorstehenden Ansprüche, dadurch gekennzeichnet, daß in einem ersten Schritt die erforderliche Menge Polyvinylpyrrolidon in Wasser gelöst und über einen Zeitraum von wenigstens 15 min bei einer Temperatur von mehr als 115°C autoklaviert wird, diese Lösung nach dem Abkühlen in einem zweiten Schritt mit N-Methylpyrrolidon und/oder 2-Pyrrolidon, Stabilisierungsmittel und einer Magnesiumverbindung, jeweils in den erforderlichen Mengen, versetzt und bis zur Homogenität gerührt wird und danach in einem dritten Schritt die erforderliche Menge Oxytetracyclin, Doxycyclin oder eines Derivats davon eingerührt wird, wobei der gewünschte pH-Wert durch Zugabe einer organischen Base eingestellt wird.

17. Verfahren nach Anspruch 16, dadurch gekennzeichnet, daß die zur Einstellung des pH-Wertes erforderliche Base der autoklavierten Lösung vor Zugabe der Magnesiumverbindung und des Oxytetracyclins, Doxycyclins oder Derivats davon zugesetzt wird.

18. Verfahren nach Anspruch 16 oder 17, dadurch gekennzeichnet, daß die Einstellung des pH-Wertes im wesentlichen mit einer basischen Aminosäure erfolgt.

19. Verfahren nach einem der Ansprüche 16 bis 18, dadurch gekennzeichnet, daß die eingesetzten Ausgangsmaterialien im wesentlichen chloridionenfrei sind.
